# EUROPÄISCHE PATENTSCHRIFT

(11) **EP 0 864 637 B1**
(45) Veröffentlichungstag und Bekanntmachung des Hinweises auf die Patenterteilung: **05.02.2003**
(21) Anmeldenummer: 98104728.5
(22) Anmeldetag: 16.03.1998
(51) Int. Cl.: C11D 1/00, C11D 17/00, A61L 9/04

(54) **Gelbasierter Reinigungsblock für die Toilettenhygiene mit permanenter Raumluftbeduftung**
Gel-bases cleansing block for lavatory hygiene with permanent air scenting
Bloc nettoyant à base de gel pour une hygiène des toilettes avec de l'air parfumé en permanence

(30) Priorität: 14.03.1997 DE 19710635
(43) Veröffentlichungstag der Anmeldung: 16.09.1998
(73) Patentinhaber: Buck-Chemie GmbH ., 71083 Herrenberg (DE)
(72) Erfinder: Dettinger, Johannes Dr., 72160 Horb (DE); Jaeschke, Edgar, 70794 Filderstadt (DE); Seidel, Detlef, 73734 Esslingen (DE)
(74) Vertreter: Mammel, Ulrike

(56) Entgegenhaltungen:
- GB-A- 2 061 996
- GB-A- 2 288 186
- US-A- 3 767 787
- US-A- 4 666 671

## Beschreibung

Die Erfindung betrifft ein kombiniertes Toilettenreinigungs- und Permanent-Raumlufterfrischungsmittel sowie ein Verfahren zu seiner Herstellung.

Toilettenreinigungsmittel, die gleichzeitig als Raumlufterfrischungsmittel durch Beduftung des Toilettenbereiches dienen, sind im Stand der Technik bekannt. Diese werden in der Regel an einem Halter oder in einem Korb oder käfigartigen Behälter im Toilettenbecken an einer Stelle angebracht, die bei jedem Spülvorgang von zulaufendem Spülwasser durchströmt wird.

Diese bekannten Toilettenreinigungsmittel zeichnen sich dadurch aus, daß sie ausschließlich durch den Spülstrom des Spülwassers der Toilette verbraucht werden, wobei sie Tenside sowie andere Bestandteile freisetzen. Die Lebensdauer dieser Toilettenreinigungsmittel wird daher durch die Anzahl der Spülungen in den Toiletten, in deren Toilettenbecken sie angebracht sind, bestimmt. Diese erzielte Lebensdauer, die Reinigungswirkung aber auch der Gehalt an Duftstoffen für die Raumbeduftung sind dabei regelmäßig an dem Gebrauch durch eine 3-bis 4-köpfige Familie orientiert, so daß eine ausreichende Raumbeduftung dann nicht mehr stattfinden kann, wenn der Haushalt oder der Ort, in deren Bereich die das Reinigungsmittel enthaltende Toilette sich befindet, von weniger Personen und/oder nicht dauerhaft benutzt wird. Gleichzeitig sorgen die in solchen Reinigungsmitteln häufig enthaltenen nichtionischen Tenside dafür, daß die Duftstoffe mehr oder weniger zurückgehalten werden und nicht in die Umgebung des Toilettenbereiches verdampfen können.

Um eine intensive Raumbeduftung zu erzielen, müßten daher große Mengen an Duftstoffen eingearbeitet werden, was im Hinblick auf ökonomische Überlegungen nachteilig ist. Gleichzeitig würde ein großer Teil dieser Duftstoffe durch den Spülstrom zusammen mit den Reinigungsmitteln, an denen sie anhaften, wirkungslos in die Kanalisation gespült.

Aus dem US-Patent 4,666,671 sind Raumerfrischungsblocks für den Toilettenbereich bekannt, die Gelbildner, Duftstoffe sowie Lösungsmittel enthalten. Mit diesen in Gelform vorliegenden Raumerfrischungsblocks kann zwar auch bei wenig oder nicht dauerhaft benutzten Toiletten eine konstante Raumbeduftung erreicht werden. Diese Blocks dienen jedoch ausschließlich der Raumerfrischung des Toilettenbereichs. Da sich die aus dem US-Patent bekannten Raumerfrischungsblocks nicht im Spülwasserstrom nach und nach auflösen, kann durch diese Blocks keine Reinigungswirkung erreicht werden. Auch eine Schaumbildung wird bei diesen Sticks nicht beobachtet, die der Verbraucher ja als optische Anzeige des Reinigungsvorgangs wünscht.

Um die gewünschte Reinigungswirkung zu entfalten und die Tenside sowie weitere Substanzen nach und nach freizusetzen, ist es natürlich erforderlich, daß sich der Block nach einer gewissen Anzahl von Spülvorgängen aufgelöst hat. Die Auflösung des Blocks ist auch erforderlich, da sie dem Verbraucher als Anzeige dafür dient, daß der Block verbraucht und durch einen neuen zu ersetzen ist. Ungenügend sind somit auch solche Blocks, bei denen zwar eine gewisse Menge an Tensiden oder anderen Komponenten durch die Wasserspülung in Lösung geht, jedoch ein erheblicher Teil des Blocks als unlöslicher, nicht reinigender Rest in dem Behältnis am Toilettenrand verbleibt: Der Verbraucher weiß somit nicht, wann der Block zu wechsein ist, es findet keine Reinigung mehr statt und der Verbraucher muß diesen Rest auch noch entsorgen.

Der Erfindung liegt daher die Aufgabe zugrunde, ein Mittel für den Toilettenbereich anzugeben, das bei nur geringer Duftstoffdosierung eine-ausreichende und-permanente Raumbeduftung auch dann ermöglicht, wenn keine Toilettenspülung erfolgt, gleichzeitig den Toilettenbereich reinigt, schäumt und sich nach ca. 200 bis 400 Spülzyklen aufgelöst hat. Selbstverständlich muß ein Toilettenreinigungsblock auch eine hinreichend hohen Erstarrungspunkt aufweisen, damit er nicht "davonfließt".

Diese Aufgabe wird durch die Merkmale des Anspruchs 1 gelöst.

Unter Lyogel wird ein an Flüssigkeit reiches, disperses System aus mindestens zwei Komponenten verstanden, nämlich einem festen, kolloid zerteilten Gelbildner und einer Flüssigkeit als Dispersionsmittel.

Das erfindungsgemäße Toilettenreinigungsmittel zeichnet sich durch ein definiertes Abdampfprofil aus, was zu einer permanenten Raumbeduftung führt. Im Gegensatz zu den bekannten stückförmigen Toilettenreinigungsmitteln hat das erfindungsgemäße Mittel eher eine gallertartige, insbesondere gelartige Konsistenz, ohne dabei seine Form zu verlieren. Diese besonderen Eigenschaften werden dem erfindungsgemäßen Mittel durch das Zusammenwirken von Gelbildner und Lösungsmittel verliehen. Durch diese besondere Konsistenz des Reinigungsmittels wird die permanente Raumbeduftung ermöglicht, weil nunmehr das Duftmittel auch ohne Spülvorgang abdampfen kann. Das Toilettenreinigungsmittel weist einen neuartigen optischen Eindruck auf und kann insbesondere durchscheinend oder optisch vollständig klar sein. Des weiteren ist ein zeitgesteuerter und gebrauchsgesteuerter Verbrauch erreichbar.

Durch die Anwesenheit der Tenside in Kombination mit den erfindungsgemäßen Abspülregulatoren wird weiterhin erreicht, daß ein gewisser Anteil des Blocks bei jedem Spülvorgang in Lösung geht, so daß die reinigenden Komponenten in der benötigten Menge kontinuierlich freigesetzt werden und sich der Block nach und nach unter Schaumbildung vollständig auflöst.

Die als Lyogele vorliegenden Toilettenreinigungsmittel weisen im allgemeinen zwischen 10 und 70 Gew.% an Lösungsmittel auf, bevorzugt sind zwischen 40 und 60 Gew.%, und besonders bevorzugt zwischen 45 und 55 Gew.%. Unter Lösungsmittel werden prinzipiell alle flüssigen Substanzen verstanden, die mit den Gelbildnern Lyogele bilden. Da die erfindungsgemäßen Abspülregulatoren ebenfalls flüssig sind und in Verbindung mit den Gelbildnern Lyogele bilden können, sind die Abspülregulatoren häufig gleichzeitig Lösungsmittel.

Als Lösungsmittel kann zumindest teilweise Wasser dienen, was den Vorteil hat, daß ein äußerst preisgünstiges und zugleich umweltfreundliches Lösungsmittel Verwendung finden kann. Wasser kann in einer Konzentration zwischen 10 und 70 Gew. %, vorzugsweise zwischen 5 und 15 Gew.% eingesetzt werden.

Als Lösungsmittel oder als Bestandteil eines Lösungsmittelgemischs sind auch flüssige, wenigstens eine Hydroxygruppe umfassende organische Lösungsmittel, vorzugsweise aus der Gruppe der ein- und/oder mehrwertigen Alkohole sowie deren Alkylether, geeignet. Besonders bevorzugt ist, daß die Abspülregulatoren selbst als Lösungsmittel dienen.

1,2 Propylenglykol wird vorzugsweise in einem Konzentrationsbereich von 11 bis 70 Gew.%, besonders bevorzugt zwischen 40 und 50 Gew.%, eingesetzt, Propylenglykol-n-Butylether vorzugsweise zwischen 10 und 70 Gew.%, bevorzugt zwischen 5 und 20 Gew.%, besonders bevorzugt zwischen 10 und 15 Gew.% und Dipropylenglykol-Methylether vorzugsweise zwischen 10 und 70 Gew.%, besonders bevorzugt zwischen 30 und 40 Gew.% eingesetzt.

Es wurde festgestellt, daß 1,2-Dipropylenglykol und Wasser als Lösungsmittel bzw. Lösungsmittelbestandteil den Erstarrungspunkt des Toilettenreinigungsblocks herabsetzen, wohingegen der Erstarrungspunkt durch Zusatz von Dipropylenglykol-Methylether erhöht werden kann. Weiterhin wurde festgestellt, daß Dipropylenglykol-Methylether und/oder Propylenglykol-n-Butylether sich günstig auf die gewünschte Freisetzung der Duftstoffe an Luft auswirken können.

Propylenglykol-n-Butylether dient als besonders bevorzugter Abspülregulator für die Auflösegeschwindigkeit: Höhere Konzentrationen an Propylenglykol-n-Butylether bewirken eine beschleunigte Auflösung des Toilettenreinigungsblocks, die beispielsweise dann gewünscht ist, wenn die den Toilettenreinigungsblock bei einem Spülvorgang umspülende Wassermenge relativ gering ist, beispielsweise, weil der Block in eine Schale gegossen und mit dieser Schale an der Toilettenschüssel eingehängt ist.

Eine erste bevorzugte Ausführungsform betrifft einen Toilettenreinigungsblock, der wegen seines vergleichsweise geringen Erstarrungspunktes direkt in eine Schale gegossen und mit dieser Schale in der WC-Schüssel eingehängt wird. Da ein solcher Toilettenreinigungsblock bei einem Spülvorgang mit einer vergleichsweise geringen Wassermenge umspült wird, hat es sich als günstig erwiesen, als Lösungsmittel-Abspülregulator-Gemisch 1,2 Propandiol, Propylenglykol-n-Butylether und Wasser zu verwenden, wobei letzteres bereits in den üblicherweise eingesetzten Ausgangsmaterialien in der erforderlichen Menge enthalten sein kann. Durch den Propylenglykol-n-Butylether verdampft der Block unter Freisetzung der Duftstoffe an Luft, und gleichzeitig wird die gewünschte Auflösegeschwindigkeit in Wasser erreicht.

In einer zweiten bevorzugten Ausführungsform werden als Lösungsmittel und/oder Abspülregulator Propylenglykol-n-Butylether und Dipropylenglykol-Methylether gemeinsam verwendet. Ein weiterer Lösungsmittelbestandteil kann Wasser sein, das meist in den eingesetzten Ausgangsstoffen bereits enthalten ist. Der Zusatz von Dipropylenglykol-Methylether bewirkt zum einen eine Erhöhung des Erstarrungspunktes des Toilettenreinigungsblocks und hat zum anderen eine günstige Auswirkung auf das gewünschte Abdampfprofil. Bei höheren Konzentrationen an Dipropylenglykol-Methylether kommt es zur zunehmenden Eintrübung des Toilettenreinigungsblocks, was jedoch unproblematisch ist, solange der Block sowohl beim Stehenlassen an der Luft als auch beim Abspülen noch die gewünschte Menge an Duftstoff freisetzt.

Durch den Zusatz von Propylenglykol-Methylether weisen diese Blocks gemäß der zweiten bevorzugten Ausführungsform einen höheren Erstarrungspunkt auf, so daß sie üblicherweise in Formen gegossen werden, wo sie erstarren, anschließend entformt werden und in einen klassischen WC-Korb überführt werden.

Die Duftstoffe können in Konzentrationen ab 0,5 Gew.%, vorzugsweise zwischen 2 und 20 Gew.%, eingesetzt werden, wobei ein Konzentrationsbereich zwischen 3 und 10 Gew.% besonders bevorzugt ist. Die Höhe der Duftstoffkonzentration hängt selbstverständlich von der Intensität der gewünschten Raumbeduftung ab. Durch das Vorliegen des Toilettenreinigungsblocks als Lyogel kann der Zusatz an Duftstoffen bei der gewünschten permanenten Raumbeduftung in einem wirtschaftlich günstigen Rahmen gehalten werden.

Bei den Duftstoffen sollte es sich um Duftstoffkompositionen, Einzelriechstoffe oder Parfüms handeln, deren Zusammensetzung das Gelieren zu einem klaren, synäresefreien Block nicht behindert. Als geeignete Duftstoffkompositionen haben sich als besonders geeignet erwiesen "Citro Mint 5438 PV" der Fa. Curt Georgi, Böblingen, "Melon GE 97.8215" der Fa. Quest International, Hamburg und "Rain Man D 60983 LF" der Fa. Haarmann & Reimer, Holzminden.

Da die Parfümkomposition mit dem Gelbildner, insbesondere der Metallseife, in der Regel kein Lyogel bildet, sind unter Lösungsmittel im Sinne der Erfindung keine Parfümkompositionen oder Parfümöle zu verstehen. Die Verwendung von Parfümkompositionen oder Parfümölen als Lösungsmittel des Lyogels scheidet darüber hinaus auch wegen des hohen Gewichtsanteils an Lösungsmitteln aus Kostengründen aus.

Durch den erfindungsgemäßen Toilettenreinigungsblock als Lyogel können die gewünschten Duftstoffe selbst in Konzentrationen bis zu 20 Gew.% zugesetzt werden, was bei dem nicht als Lyogel vorliegenden herkömmlichen Toilettenreinigungsblock infolge Weichwerdens nicht möglich ist.

Als erfindungsgemäße Gelbildner werden vorzugsweise Metallseifen eingesetzt. Dies sind vor allem die Alkalisalze der höheren Fettsäuren, insbesondere die Natriumsalze, beispielsweise Natriumstearat. Prinzipiell sollte die Konzentration an Gelbildnern zwischen 2,5 Gew.% und 20 Gew.% betragen, wobei sich für die Metallseifen und insbesondere für das Natriumstearat eine Konzentration zwischen 3 Gew.% und 10 Gew.% bewährt hat.

Um die gewünschte Lyogelbildung zu erreichen, muß das Konzentrationsverhältnis von Gelbildner zu Lösungsmittel natürlich aufeinander abgestimmt sein. Je nach verwendetem Gelbildner und Lösungsmittel(gemisch) sind prinzipiell Konzentrationsverhältnisse zwischen 1:4 und 1:12 möglich, vorzugsweise beträgt das Konzentrationsverhältnis zwischen 1:5 und 1:9.

Weitere Bestandteile des erfindungsgemäßen Toilettenreinigungsmittels können anionische Tenside, nichtionische Tenside, Farbstoffe, Amphotenside, Konservierungsmittel und /oder Komplexierungsmittel sein.

In einer bevorzugten Ausführungsform besteht das erfindungsgemäße Toilettenreinigungsmittel aus in etwa 5 bis 25 Gew.% anionischem Tensid a). Dabei finden insbesondere solche, ausgewählt aus der Gruppe bestehend aus Alkylsulfat, Fettalkoholsulfat, Fettalkoholethersulfat, Olefinsulfonat und Alkylbenzolsulfonat oder deren Mischungen, Verwendung. Dabei sind Alkylkettenlängen der Alkylreste oder Fettsäurebestandteile von C₈ - C₁₈ bevorzugt. Das erfindungsgemäße Toilettenreinigungsmittel zeigt bei jedem Spülvorgang eine Schaumentwicklung, die von der Art und Menge des verwendeten anionischen Tensids abhängig ist. Diese Schaumentwicklung hat den Vorteil, daß dem Anwender die Reinigungswirkung des erfindungsgemäßen Reinigungsmittels durch die Schaumentwicklung sichtbar gemacht wird. Eine Reinigungswirkung wird insbesondere durch die nichtionischen Tenside und in Kombination durch die Lösungsmittel erreicht.

Des weiteren sind 1 bis 10 Gew.% Amphotensid, insbesondere in Form von Betain, in dem Toilettenreinigungsmittel zugegen.

Wahlweise können bis zu 25 Gew.% nichtionisches Tensid in dem Toilettenreinigungsmittel enthalten sein. Hier eignen sich besonders die Anlagerungsprodukte von 1 bis 80 Mol Ethylenoxid an 1 Mol einer aliphatischen Verbindung mit im wesentlichen 8 bis 20 Kohlenstoffatomen aus der Gruppe der Alkohole, Fettsäuren und Fettsäureamide. Möglich ist auch der Einsatz von Aminoxiden sowie von Kondensationsprodukten aus primären Alkoholen und Glukose sowie Lactobionsäure und deren Derivate.

Als weiteren wahlweisen Bestandteil kann das erfindungsgemäße Toilettenreinigungsmittel ein Konservierungsmittel in einer Menge von bis zu 1 Gew.% enthalten.

Außerdem können bis zu 20 Gew.% weitere Zusätze enthalten sein. Dies können Desinfektionsmittel, Bleichmittel, Aktivatoren, Farbe sowie Kalk und Urinstein lösende Säuren sein, wobei diese Aufzählung nicht abschließend ist.

Der erfindungsgemäße Toilettenreinigungsmittelblock ist vorzugsweise frei von chlorabspaltenden Substanzen.

Das erfindungsgemäße Toilettenreinigungsmittel weist üblicherweise die folgende Zusammensetzung auf:

| | | |
|---|---|---|
| a) | 5 - 25 Gew.% | anionischem Tensid, |
| b) | 2,5 - 15 Gew.% | Metallseife, |
| c) | 10 - 70 Gew.% | Lösungsmittel, |
| d) | 1 - 10 Gew.% | Amphotensid, insbesondere Betain, |
| e) | 0,5 - 10 Gew.% | Duftstoffen, |
| f) | 0 - 25 Gew.% | nichtionischem Tensid, |
| g) | 0 - 1 Gew.% | Konservierungsmittel und |
| h) | 0 - 20 Gew.% | weiteren Zusätzen, wie Komplexbildner, Farbstoffe etc., |

wobei ein Toilettenreinigungsmittel mit folgender Zusammensetzung bevorzugt ist:

| | | |
|---|---|---|
| a) | 5 - 25 Gew.% | anionischem Tensid, ausgewählt aus der Gruppe bestehend aus Alkylsulfat, Fettalkoholsulfat, Fettalkoholethersulfat, Olefinsulfonat und Alkylbenzolsulfonat oder deren Mischungen, |
| b) | 2,5 - 10 Gew.% | Metallseife, ausgewählt aus den Alkalisalzen der höheren Fettsäuren, |
| c) | 10 - 60 Gew.% | Lösungsmittel, ausgewählt aus der Gruppe bestehend aus einwertigen und mehrwertigen Alkoholen, wie Ethanol und Glykolen, Polyglykolen sowie Alkylethern der Glykole, |
| d) | 1 - 10 Gew.% | Amphotensid, |
| e) | 1 - 5 Gew.% | Duftstoffen, |
| f) | 0 - 25 Gew.% | nichtionischem Tensid, ausgewählt aus der Gruppe bestehend aus den Anlagerungsprodukten von 1 - 80 mol Ethylenoxid an 1 Mol einer aliphatischen Verbindung mit 8 - 20 C Atomen aus der Gruppe der Alkohole, Fettsäuren und Fettsäureamide, der Kondensationsprodukte aus primären Alkoholen und Glukose, Aminoxide sowie der Lactobionsäure und deren Derivate, |
| g) | 0 - 1 Gew.% | Konservierungsmittel und |
| h) | 0 - 20 Gew.% | weiteren Zusätzen. |

Die Erfindung betrifft auch ein Verfahren zur Herstellung der oben genannten Toilettenreinigungsmittel. Diese werden dadurch hergestellt, daß die das fertige Reinigungsmittel bildenden Bestandteile, nämlich Tenside, Gelbildner, Duftstoffe sowie die weiteren Komponenten mit dem Lösungsmittel und dem Abspülregulator unter Erwärmen solange gerührt werden, bis eine im wesentlichen klare Lösung entsteht. Bei der Verwendung einer Metallseife, z. B. Natriumstearat als Gelbildner, den erfindungsgemäßen Abspülregulatoren und Wasser als Lösungsmittel hat sich das Rühren der Bestandteile bei einer Temperatur von etwa 80 °C bis 90 °C bewährt. Das Rühren erfolgt vorzugsweise in einem Rührkessel. Die jeweils geeignete Erwärmungstemperatur hängt von den ausgewählten Einzelkomponenten, deren Konzentrationen und dem oder den Lösungsmitteln ab. Dann wird die erhaltene Lösung in Formen oder Schalen gegossen, wo sie zu den stückförmigen Toilettenreinigungsmitteln erstarrt.

Üblicherweise wird das erfindungsgemäße Toilettenreinigungsmittel an einem Halter oder in einem Korb oder käfigartigen Behälter im Toilettenbecken an einer Stelle angebracht, die bei jedem Spülvorgang vom zulaufenden Spülwasser durchströmt wird. Infolge des Vorliegens als Lyogel findet ein Verbrauch des erfindungsgemäßen Reinigungsmittels auch ohne Betätigung des Spülvorgangs an der Luft statt, so daß auch ohne Spülwasserzufluß eine permanente Beduftung des Toilettenbereichs erreicht wird.

### Ausführungsbeispiele und Vergleichsversuche

### Erfindungsgemäße Ausführungsbeispiele: I.1 bis II.2

Die Rezepturen I.1 und I.2 betreffen jeweils einen Toilettenreinigungsblock gemäß der ersten und die Rezepturen II.1 und II.2 gemäß der zweiten bevorzugten Ausführungsform, nachfolgend in Tabelle 1a und 1b dargestellt, wobei für beide dieser Ausführungsformen jeweils eine Rezeptur mit einer Parfümkonzentration von 3 Gew.% (.1) und einer Parfümkonzentration von 10 Gew.% (.2) angegeben ist.

Der gewichtsprozentuale Anteil an Wasser in den Rezepturen stammt in den Rezepturen I.1. und II.1 ausschließlich aus dem Wassergehalt der eingesetzten Komponenten, in den Rezepturen I.2 und II.2 wurde zusätzlich Wasser als Lösungsmittel zugesetzt.

In Tabelle 1a sind die Zusammensetzungen der vier verschiedenen Ausführungsbeispiele in bezug auf die einzelnen Substanzen aufgeführt:

**Tabelle 1a:**

| **Ausführungsbeispiel** | **I.1** | **I.2** | **II.1** | **II.2** |
|---|---|---|---|---|
| Wasser | 8,48 | 9,48 | 8 | 13 |
| 1,2 Propandiol | 47,10 | 40,10 | 0,10 | 0,00 |
| Propylenglykol-n-Butylether | 11,00 | 5,00 | 18,00 | 11,00 |
| Dipropylenglykol-Methylether | | | 40,00 | 34,00 |
| Nariumstearat, wasserfrei (ca. 1% Wasser) | 7,92 | 7,92 | 7,92 | 7,92 |
| Betain (ca. 44%ig) | 2,20 | 2,20 | 2,20 | 2,20 |
| Betain | | 5,00 | | 4,00 |
| Kokosfettsäuremonoethanolamid | 5,00 | 5,00 | 5,00 | 5,00 |
| Natriumlaurylethersulfat (2EO) ca. 70%ig | 13,30 | 13,30 | 13,30 | 12,60 |
| Parahydroxybenzoesäuremethylester | 0,08 | 0,08 | 0,08 | 0,08 |
| Parahydroxybenzoesäurepropylester | 0,12 | 0,12 | 0,12 | 0,12 |
| 90% Hydriertes Ricinusölethoxylat mit 40 Mol EO | 1,80 | 1,80 | 1,80 | 0,00 |
| Parfum | 3,00 | 10,00 | 3,00 | 10,00 |
| Summe | 100 | 100 | 100 | 100 |

Als 1,2 Propandiol wurde 1,2 Propylenglykol der Firma BASF verwendet. Propylenglykol-n-Butylether wurde als Dowanol PnB, Dipropylenglykol-Methylether als Dowanol DPM der Firma DOW eingesetzt. Von der Firma Bährlocher wurde das Natriumstearat eingesetzt. Als 44%iges Betain wurde Rewoteric AMB 50 und als Betain wurde Rewoteric AMB 12 von Witco verwendet. Als Kokosfettsäuremonoethanolamid wurde Comperlan 100 von Henkel, als Natriumlaurylethersulfat (2EO) ca. 70%ig wurde Texapon N 70 von Henkel eingesetzt. Parahydroxybenzoesäuremethylester wurde sowohl als Solbrol M, als auch als Solbrol P der Firma BAYER verwendet. Das eingesetzte Chremophor RH 455 von BASF besteht zu 90 % aus hydriertem Ricinusölethoxylat mit 40 Mol EO, zu 5 % aus 1,2 Propylenglykol und zu 5 % aus Wasser.

In Tabelle 1b sind die Rezepturen der vier Ausführungsbeispiele in bezug auf die eingesetzten Ausgangsstoffe zusammengestellt.

**Tabelle 1b:**

| **Ausführungsbeispiel:** | **I.1.** | **I.2.** | **II.1.** | **II.2.** |
|---|---|---|---|---|
| Wasser | 0 | 1 | 0 | 5 |
| 1,2 Propylenglykol | 47 | 40 | 0 | 0 |
| Dowanol PnB | 11 | 5 | 18 | 11 |
| Dowanol DPM | | | 40 | 34 |
| Natriumstearat | 8 | 8 | 8 | 8 |
| Rewoteric AMB 50 | 5 | 5 | 5 | 5 |
| Rewoteric AMB 12 | | 5 | | 4 |
| Comperlan 100 | 5 | 5 | 5 | 5 |
| Texapon N 70 | 19 | 19 | 19 | 18 |
| Solbrol M | 0,08 | 0,08 | 0,08 | 0,08 |
| Solbrol P | 0,12 | 0,12 | 0,12 | 0,12 |
| Chremophor RH 455 | 2 | 2 | 2 | 0 |
| Parfüm | 3 | 10 | 3 | 10 |
| Summe | 100 | 100 | 100 | 100 |

Falls ein noch intensiverer Duft erwünscht ist, kann die Parfümkonzentration ohne Beeinträchtigung der Konsistenz des Toilettenreinigungsblocks noch weiter bis auf etwa 20 Gew.% erhöht werden.

Die Rezeptur des als Vergleichsversuch dienenden "klassischen" Toilettenreinigungsblocks ist in Tabelle 2 angegeben.

**Tabelle 2:**

| **Ausführungsbeispiel:** | **klassisch**: |
|---|---|
| | LAS |
| sec. N-Alkyl-(C10-13)-benzolsulfonat, Na-Salz | 15-25% |
| Natriumchlorid | 15-25% |
| Natriumsulfat, calz. | 5-15% |
| Alkyl-(C13/15)-polyethylenglykolether (5 Mol EO) | 2-7% |
| Alkyl-(C16-18)-polyethylenglykolether (25 Mol EO) | 1-6% |
| Farbstoff | 0-1% |
| Titandioxid | 0-1% |
| Parfüm | 3-8% |
| N-Alkyl-(C12-14)-sulfat, Na-Salz | |
| Alkylpolyglykosid | |

In der nachfolgenden Tabelle 3 sind die Eigenschaften der Toilettenreinigungsblocks mit permanenter Raumbeduftung denen eines "klassischen" Toilettenreinigungsblocks gegenübergestellt:

**Tabelle 3:**

| Ausführungsbeispiel: | **I.1.** | **I.2.** | **II.1.** | **II.1.** | klassisch: | |
|---|---|---|---|---|---|---|
| Erscheinungsbild | **fest/fast klar** | **fest/ klar** | **fest/ leicht trüb** | **fest/ klar** | | |
| Schaumzahl | 140/135/130 | | 130/125/110 | 90/80/60 | 110/100/90 | 80/60/40 |
| nach DIN Spülzahl [Anzahl | 135 | 240 | 210 | 220 | 300-400 | 300-400 |
| Spülungen] (Mittelwert): Netzzahl | | | 126 | 100 | 30 | 110 |
| nach DIN Erstarrungspunkt (°C) | 42 | 39 | 73 | 49 | | |

Die erfindungsgemäßen Toilettenreinigungsblocks zeichnen sich durch gute Schaum-, Spül- und Netzzahlen aus.

Zur Untersuchung der gewünschten permanenten Raumluftbeduftung wurden die Toilettenreinigungsblöcke eines Anfangsgewichts von ca. 30 g an Luft über einen Zeitraum von bis zu 33 Tagen gelagert und die Gewichtsabnahme registriert. Die Meßwerte sind in der nachfolgenden Abbildung 1 dargestellt:

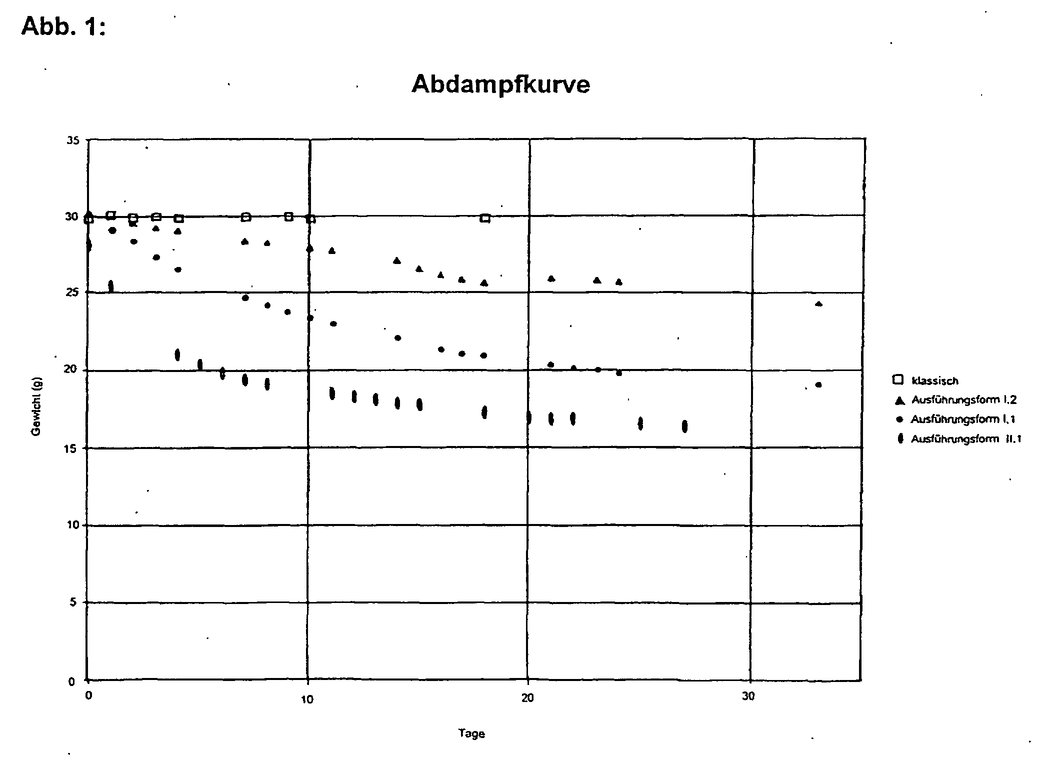

Die Gewichtsabnahme der erfindungsgemäßen Reinigungsblocks zeigt, daß diese auch an Luft kontinuierlich Duftstoffe freisetzen, wohingegen der klassische Toilettenreinigungsblock keine Gewichtsabnahme an Luft und somit keine Duftstoff-Freisetzung zeigte.

Die als Lyogele vorliegenden Toilettenreinigungsblocks gemäß den Rezepturen I.1 bis II.2 zeichnen sich somit auch durch eine permanente Raumbeduftung gegenüber den bekannten Toilettenreinigungsblöcken aus.

### Vergleichsversuche:

1. Raumlufterfrischungs-Blocks gemäß dem US-Patent 4,666,671 bestehend aus 5 Gew.% Wasser, 34 Gew.% 2-(2-Ethoxyethoxy)ethanol, 4 Gew.% Natriumstearat, 20 Gew.% Propanol, 35 Gew.% Parfümöl und 2 Gew.% Germiciden zeigten zwar einen von der Anzahl der Spülcyclen unabhängigen konstanten Duft, die Blocks lösten sich jedoch auch nach mehr als 400 Spülcyclen überhaupt nicht auf, vielmehr nahm ihr Gewicht durch Einlagerung von Wasser sogar zu.
2. Dem Block gemäß Vergleichsversuch 1 wurden Tenside zugesetzt. Ein "kontinuierlicher" Auflösevorgang konnte bei diesen Toilettenblocks nicht beobachtet werden. Nachdem anfänglich Tenside freigesetzt wurden und die gewünschte Schaumbildung stattfand, wurde keine weitere Auflösung beobachtet und trotz des visuellen Anscheins eines noch wirksamen Reinigungsblocks fand keine Reinigung mehr statt.
3. Um das Abspülverhalten zu verbessern, wurden dem Block aus Vergleichsversuch 2 die üblichen Abspülregulatoren Dipropylenglykol, Natriumbutylmonoglycolsulfat, Natriumcumolsulfonat und Natriumtoluolsulfonat zugesetzt. Auch diese Blocks zeigten nicht das gewünschte Abspülverhalten, da nach einer anfänglichen Auflösung der Block "verbackte" und der Auflösevorgang beendet war.
4. Ein nur aus Wasser und 1,2 Dipropylenglykol als Lösungsmittel, Natriumstearat als Gelbildner, Duftstoffen sowie den weiteren üblichen Bestandteilen hergestellten Toilettenreinigungsblock ohne Zusatz von Propylenglykol-n-Butylether oder Dipropylenglykol-Methylether löste sich auch bei sehr hohen Spülzahlen nicht auf.

## Patentansprüche

1. Permanent die Raumluft erfrischendes Toilettenreinigungsmittel in Stückform, **dadurch gekennzeichnet, dass** es Tenside, Abspülregulatoren, Gelbildner, Duftstoff sowie Lösungsmittel enthält und als Lyogel vorliegt, wobei der Abspülregulator ausgewählt wird aus der Gruppe der ein-, mehrwertigen Alkohole und/oder deren Alkylether, das Mittel insbesondere zwischen 0,5 und 20 Gew.% Duftstoffe umfasst und ein gewisser Teil des Mittels bei jedem Spülvorgang in Lösung geht, so dass die reinigenden Komponenten in der benötigten Menge kontinuierlich freigesetzt werden und sich der Block nach und nach unter Schaumbildung auflöst.

2. Toilettenreinigungsmittel nach Anspruch 1, **dadurch gekennzeichnet, dass** der Abspülregulator, 1,2 Propylenglykol, Polyglykol, Propylenglykol-nbutylether oder Dipropylenglykol-Methylether ist.

3. Toilettenreinigungsmittel nach Anspruch 1 oder 2, **dadurch gekennzeichnet, dass** der Gelbildner eine Metallseife ist, welche Metallseife vorzugsweise aus der Gruppe der Alkaliseifen der höheren Fettsäuren ausgewählt wird und besonders bevorzugt Natriumstearat ist.

4. Toilettenreinigungsmittel nach Anspruch 2 oder 3, **dadurch gekennzeichnet, dass** das Lösungsmittel wenigstens teilweise Wasser und/oder ein flüssiges, wenigstens eine Hydroxygruppe umfassendes organisches Lösungsmittel ist.

5. Toilettenreinigungsmittel nach einem der Ansprüche 1 bis 4, **dadurch gekennzeichnet, dass** der Abspülregulator gleichzeitig wenigstens teilweise ein Lösungsmittel ist.

6. Toilettenreinigungsmittel nach einem der Ansprüche 1 bis 5, **dadurch gekennzeichnet, dass** es 2,5 bis 20 Gew.%, vorzugsweise 3 bis 10 Gew.%, Gelbildner umfasst und die Summe aus Lösungsmittel und/oder Abspülregulator 10 bis 70 Gew.%, vorzugsweise 40 bis 60 Gew.% beträgt.

7. Toilettenreinigungsmittel nach einem der Ansprüche 1 bis 6, **dadurch gekennzeichnet, dass** das Verhältnis zwischen Gelbildner und Lösungsmittel und/oder Abspülregulator zwischen 1 : 4 und 1 : 12, vorzugsweise zwischen 1 : 5 und 1 : 9, beträgt.

8. Toilettenreinigungsmittel nach einem der Ansprüche 1 bis 7, **dadurch gekennzeichnet, dass** es 5 bis 25 Gew.% anionische und/oder 0 bis 25 Gew.% nichtionische und/oder 1 bis 10 Gew.% amphotere Tenside und/oder Farbstoffe und/oder Konservierungsmittel enthält.

9. Toilettenreinigungsmittel nach einem der Ansprüche 1 bis 8, **dadurch gekennzeichnet, dass** es durchscheinend oder optisch vollständig klar ist.

10. Verfahren zur Herstellung des als Lyogel vorliegenden Toilettenreinigungsmittels nach einem der Ansprüche 1 bis 9, **dadurch gekennzeichnet, dass** Tenside, Gelbildner, Duftstoff und die weiteren Komponenten des Toilettenreinigungsmittels zusammen mit dem Lösungsmittel und/oder Abspülregulator unter Erwärmung gerührt werden, bis eine im wesentlichen klare Lösung entsteht, welche Lösung anschließend in Formen oder Schalen gegossen wird.

## Claims

1. Lavatory cleansing composition which permanently refreshes the air, in block form, **characterized in that** it comprises surfactants, flush regulators, gel formers, fragrance and solvents, and is in the form of a lyogel, where the flush regulator is chosen from the group of mono-, polyhydric alcohols and/or alkyl ethers thereof, the composition comprises, in particular, between 0.5 and 20% by weight of fragrances, and a certain fraction of the composition goes into solution during each flushing operation, such that the cleaning components are released continuously in the required amount and the block gradually dissolves with foam formation.

2. Lavatory cleansing composition according to Claim 1, **characterized in that** the flush regulator is 1,2-propylene glycol, polyglycol, propylene glycol n-butyl ether or dipropylene glycol methyl ether.

3. Lavatory cleansing composition according to Claim 1 or 2, **characterized in that** the gel former is a metal soap, which metal soap is preferably chosen from the group of alkali metal soaps of higher fatty acids and is particularly preferably sodium stearate.

4. Lavatory cleansing composition according to Claim 2 or 3, **characterized in that** the solvent is at least partially water and/or a liquid organic solvent containing at least one hydroxyl group.

5. Lavatory cleansing composition according to one of Claims 1 to 4, **characterized in that** the flush regulator is simultaneously at least partially a solvent.

6. Lavatory cleansing composition according to one of Claims 1 to 5, **characterized in that** it comprises 2.5 to 20% by weight, preferably 3 to 10% by weight, of gel former, and the sum of solvent and/or flush regulator is 10 to 70% by weight, preferably 40 to 60% by weight.

7. Lavatory cleansing composition according to one of Claims 1 to 6, **characterized in that** the ratio between gel former and solvent and/or flush regulator is between 1:4 and 1:12, preferably between 1:5 and 1:9.

8. Lavatory cleansing composition according to one of Claims 1 to 7, **characterized in that** it comprises 5 to 25% by weight of anionic and/or 0 to 25% by weight of nonionic and/or 1 to 10% by weight of amphoteric surfactants and/or dyes and/or preservatives.

9. Lavatory cleansing composition according to one of Claims 1 to 8, **characterized in that** it is transparent or optically completely clear.

10. Process for the preparation of the lavatory cleansing composition according to one of Claims 1 to 9 which is in the form of a lyogel, **characterized in that** surfactants, gel formers, fragrance and the other components of the lavatory cleansing composition are stirred together with the solvent and/or flush regulator with warming until an essentially clear solution forms, which solution is then poured into moulds or dishes.

## Revendications

1. Produit de nettoyage sous forme de bloc pour toilettes, rafraîchissant l'air ambiant, **caractérisé en ce qu'**il contient des tensioactifs, des régulateurs d'entraînement, des agents gélifiants, du parfum ainsi que des solvants et se trouve sous forme de lyogel, le régulateur d'entraînement étant choisi dans le groupe qui comprend des alcools mono-, polyhydroxylés et/ou leurs alkyléthers, le produit contenant en particulier entre 0,5 et 20 % en poids de parfums et une certaine partie du produit passant en solution à chaque chasse, de sorte que les composants nettoyants sont en continu libérés en la quantité requise et le bloc se dissout peu à peu avec formation de mousse.

2. Produit de nettoyage pour toilettes selon la revendication 1, **caractérisé en ce que** le régulateur d'entraînement est le 1,2-propylèneglycol, le polyglycol, l'éther n-butylique de propylèneglycol ou l'éther méthylique de dipropylèneglycol.

3. Produit de nettoyage pour toilettes selon la revendication 1 ou 2, **caractérisé en ce que** l'agent gélifiant est un savon métallique, lequel savon métallique est de préférence choisi dans le groupe des savons alcalins des acides gras supérieurs et de façon particulièrement préférée est le stéarate de sodium.

4. Produit de nettoyage pour toilettes selon la revendication 2 ou 3, **caractérisé en ce que** le solvant est au moins en partie l'eau et/ou un solvant organique liquide comprenant au moins un groupe hydroxy.

5. Produit de nettoyage pour toilettes selon l'une quelconque des revendications 1 à 4, **caractérisé en ce que** le régulateur d'entraînement est en même temps au moins en partie un solvant.

6. Produit de nettoyage pour toilettes selon l'une quelconque des revendications 1 à 5, **caractérisé en ce qu'**il comprend de 2,5 à 20 % en poids, de préférence de 3 à 10 % en poids, d'agent gélifiant, et la somme du solvant et/ou du régulateur d'entraînement va de 10 à 70 % en poids, de préférence de 40 à 60 % en poids.

7. Produit de nettoyage pour toilettes selon l'une quelconque des revendications 1 à 6, **caractérisé en ce que** le rapport entre agent gélifiant et solvant et/ou régulateur d'entraînement est compris entre 1:4 et 1:12, de préférence entre 1:5 et 1:9.

8. Produit de nettoyage pour toilettes selon l'une quelconque des revendications 1 à 7, **caractérisé en ce qu'**il contient de 5 à 25 % en poids de tensioactifs anioniques et/ou de 0 à 25 % en poids de tensioactifs non ioniques et/ou de 1 à 10 % en poids de tensioactifs amphotères et/ou de colorants et/ou de conservateurs.

9. Produit de nettoyage pour toilettes selon l'une quelconque des revendications 1 à 8, **caractérisé en ce qu'**il est translucide ou totalement transparent.

10. Procédé pour la fabrication du produit de nettoyage pour toilettes, se trouvant sous forme de lyogel, selon l'une quelconque des revendications 1 à 9, **caractérisé en ce qu'**on agite en chauffant les tensioactifs, l'agent gélifiant, le parfum et les autres composants du produit de nettoyage pour toilettes, conjointement avec le solvant et/ou le régulateur d'entraînement, jusqu'à l'obtention d'une solution pratiquement limpide, laquelle solution est ensuite versée dans des moules ou des capsules.
